# EUROPEAN PATENT SPECIFICATION

(11) **EP 4 346 920 B1**
(45) Date of publication and mention of the grant of the patent: **01.04.2026**
(21) Application number: 22734042.9
(22) Date of filing: 03.06.2022
(51) Int. Cl.: A61L 2/10, A61L 9/20, B64G 1/00, F21V 9/00, G02B 6/00

(54) **DISINFECTION OF A WORKING VOLUME IN AN ARTIFICIAL SPACE ENVIRONMENT VIA SOLAR UV RADIATION**
DESINFEKTION EINES ARBEITSVOLUMENS IN EINER KÜNSTLICHEN WELTRAUMUMGEBUNG MITTELS SOLARER UV-STRAHLUNG
DESINFECTION D'UN VOLUME DE TRAVAIL DANS UN ENVIRONNEMENT SPATIAL ARTIFICIEL AU MOYEN D'UN RAYONNEMENT SOLAIRE UV

(30) Priority: 04.06.2021 IT 202100014657
(43) Date of publication of application: 10.04.2024
(73) Proprietor: Istituto Nazionale di Astrofisica, 00136 Roma (IT)
(72) Inventor: ATTINA, Primo, 00136 ROMA (IT); BIANCO, Andrea, 00136 ROMA (IT); CORTECCHIA, Fausto, 00136 ROMA (IT); DIOLAITI, Emiliano, 00136 ROMA (IT); LESSIO, Luigi, 00136 ROMA (IT); LOMBINI, Matteo, 00136 ROMA (IT); MACCHI, Alberto, 00136 ROMA (IT); MALAGUTI, Giuseppe, 00136 ROMA (IT); PARESCHI, Giovanni, 00136 ROMA (IT); PELIZZO, Maria Guglielmina, 00185 ROMA (IT)
(74) Representative: Studio Torta S.p.A.
(86) International application number: PCT/IB2022/055198
(87) International publication number: WO 2022/254398

(56) References cited:
- KR-B1- 101 671 455
- RU-C1- 2 099 089

## Description

### Technical Field

The present invention relates to a device and method for disinfecting a working volume in an artificial space environment via solar UV radiation.

### Background Art

As is known, the Sun emits radiation in all the bands of the electromagnetic spectrum, with varying intensity depending on the wavelength. In particular, the intensity is greater in the intermediate region of the electromagnetic spectrum, which comprises ultraviolet (UV), visible (Vis) and infrared (IR) radiations, respectively in ascending order of wavelength (and thus in descending order of frequency).

UV radiation, also known as UV rays, comprises the wavelength range from 200 nm to 400 nm. Conventionally, three UV ray ranges are considered when analysing the effects on human health:
- UVA (so-called long-wave UV), from 400 nm to 315 nm, useful for stimulating vitamin D production in the human body, and weakly absorbed by DNA and RNA due to their relatively low frequency;
- UVB (so-called medium-wave UV), from 315 nm to 280 nm; and
- UVC (so-called short-wave UV), from 200 nm to 250 nm, whose high frequency is capable of modifying the DNA or RNA of microorganisms, enabling a germicidal action.

The visible spectrum comprises the wavelength range from about 400 nm (red colour) to 700 nm (violet colour), including all colours perceptible to the human eye. Therefore, radiation in this range of the electromagnetic spectrum is called light.

IR radiation, also known as IR rays, comprises the wavelength range from 700 nm to 1 mm. There are several conventions for classifying IR rays. In some of them, the range of IR radiation bordering the visible spectrum is called NIR ("near-infrared"), and is followed (starting from about 3500 nm) by MIR ("mid-infrared") or thermal infrared.

When crossing the Earth's atmosphere, solar radiation undergoes phenomena of reflection, refraction, absorption and diffusion, by the various atmospheric gases and to a varying extent depending on the wavelength. Therefore, the solar spectrum measured on the Earth's surface is different from that detected at the outer thresholds of the Earth's atmosphere.

In particular, the Earth's atmosphere absorbs almost all of UVC and a high percentage of UVB, thus most of the UV rays reaching the Earth's surface are UVA. Therefore, for terrestrial applications, UVC radiation is only available from artificial sources. For this purpose, germicidal lamps are known and used to produce UVC radiation to kill and/or inactivate microorganisms (including fungi and moulds).

The adoption of disinfection techniques presents even greater problems in artificial space environments, e.g. space stations, spacecraft, and lunar and planetary bases. RU 2 099 089 C1 discloses a device for disinfecting artificial space environments.

The purpose of the present invention is to realise a device for disinfecting that can be used in an artificial space environment.

### Disclosure of Invention

The aforementioned purpose is achieved by a combination of artificial space environment and device as claimed in claim 1.

The present invention also relates to a method for disinfecting as claimed in claim 14.

### Brief Description of Drawings

For a better understanding of the present invention, a preferred embodiment is described below, by way of nonlimiting example and with reference to the accompanying drawings, wherein:
- Figure 1 is a schematic perspective view of an artificial space environment comprising a device according to the present invention;
- Figure 2 is a schematic front view of a detail of Figure 1;
- Figures 3a, 3b are axial sectional views of a component of the device of Figure 1;
- Figure 4 is a perspective view of a variant of the component of Figure 3;
- Figures 5, 6 are sectional views of further variants of the component of Figure 3;
- Figures 7a, 7b, 8 are axial sectional views of components of the device of Figure 1, according to different variants;
- Figures 9a, 9b, 10 are schematic front views of variants of the device of Figure 1;
- Figure 11 is a sectional view of a component of the device of Figure 10; and
- Figure 12 is a block diagram of the control of the device of Figure 1.

### Detailed Description of the Invention

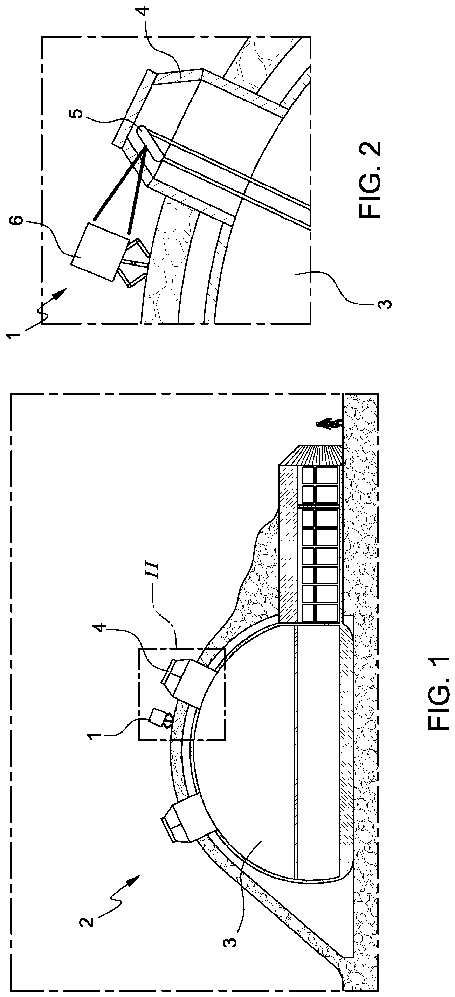

With reference to Figure 1, there is indicated by 1 a device for disinfecting according to the present invention. The device 1 is arranged externally to an artificial space environment 2. The artificial space environment 2 can be e.g. a space station, a spacecraft, a lunar base, or a planetary base (e.g. on Mars). By way of example, Figure 1 shows a schematic diagram of a lunar base 2 comprising a main environment 3, at least partially protected by compressed regolith, from which one or more domes 4 extend upwards emerging from the cover to allow observation and natural illumination. The dome 4 houses a portion of a conditioning system comprising a tank defining a working volume 5, a first conduit at the inlet of the tank for the supply of fluid to be sterilised, e.g. air coming from previous stages of a regeneration plant, and a second conduit at the outlet of the tank for the re-injection of the sterilised fluid, e.g. air re-injected into the main environment 3 or into the regeneration plant. The device 1 faces the tank through a window which is transparent to radiation of a predetermined wavelength, as described in detail hereinafter.

It should be noted that the term "working volume" is used here in its broadest sense and can comprise any closed or open volume, such as a conduit, a tank, a habitable room.

The device 1 comprises a concentrator 6 of solar radiation associated with the working volume 5, and a filter 7 coupled to the concentrator 6.

Optionally, the device 1 may comprise an optical transport means 8 of the radiation from the concentrator 6 to the at least one working volume 5 and/or a system 9 for moving the concentrator 6.

The concentrator 6 can be static or dynamic.

The static concentrators 6 are designed to receive solar radiation without requiring any movement.

The dynamic concentrators 6 are used if the direction of incidence of the solar radiation has a range of variability greater than the angle of acceptance of the concentrator 6, thus they need the system 9 for moving the concentrator 6 to be able to collect solar radiation effectively, as described below. Speed and accuracy of the movement system 9 depend mainly on where the concentrator 6 is placed. For example, at the poles of the Moon, the Sun is always visible and the direction of arrival of the solar radiation varies with the Moon's period of revolution around the Earth (about 27 days), whereas on a travelling spacecraft (e.g. to Mars) or an orbiting space station, the variation of the Sun's position with respect to concentrator 6 might vary differently.

The choice of the concentrator 6 depends on the type of application.

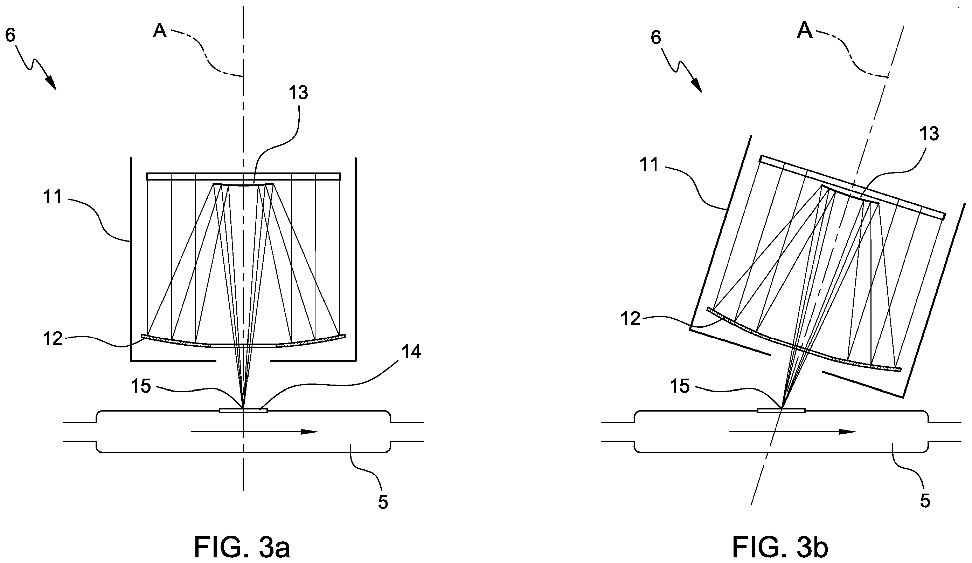

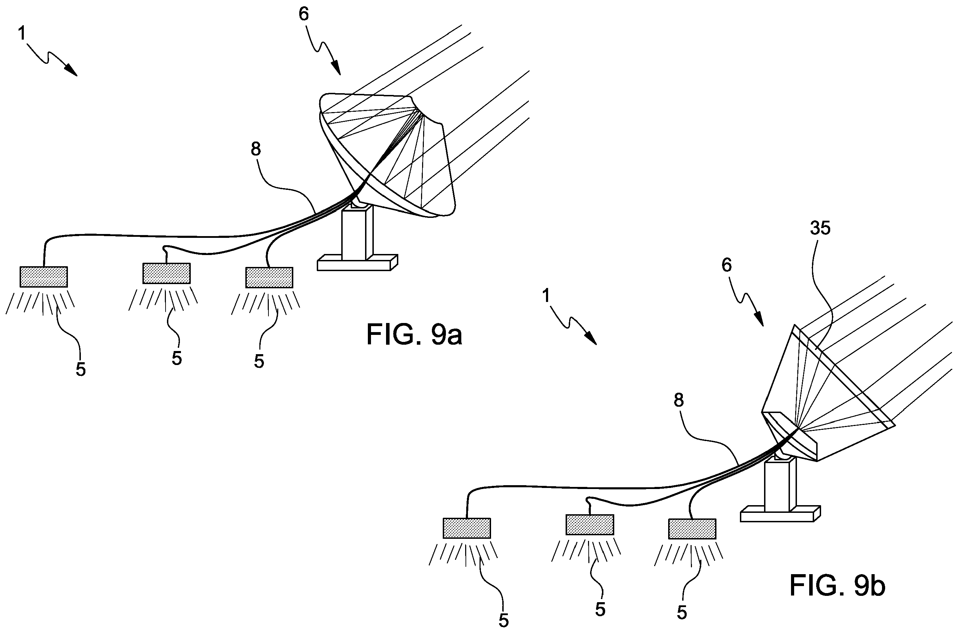

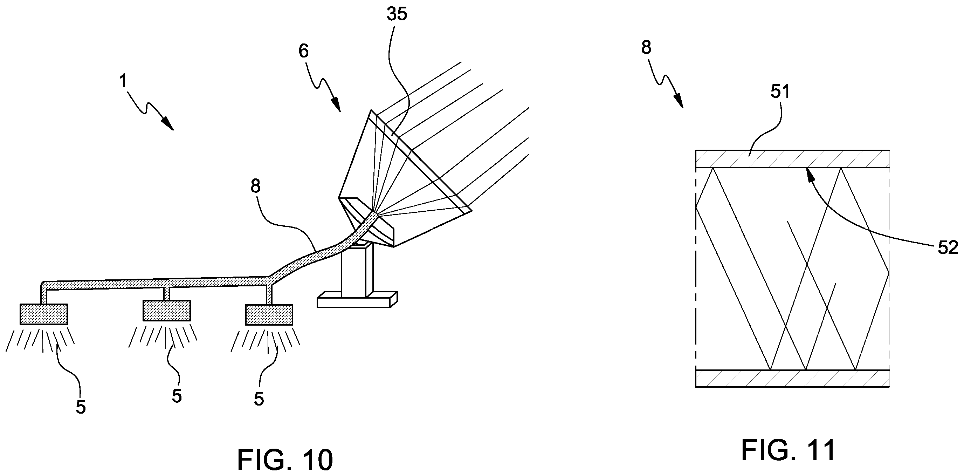

In a first embodiment (Figures 3a, 3b), the concentrator 6 is a mirror system, e.g. in a Ritchey-Chrétien optical configuration, comprising an A-axis cylindrical casing 11, a primary mirror 12 and a secondary mirror 13, e.g. hyperbolic, which are arranged within the cylindrical casing 11. The cylindrical mirror 12, of axis A, reflects the solar rays (which can be considered parallel to one another) concentrating them towards the secondary mirror 13, coaxial to the primary mirror 12 and facing it, which reflects them concentrating them in a focal plane 14, orthogonal to the axis A and passing through a point 15 of the axis A called telescope focus. The focal plane 14 can be directly inside (Figures 3a, 3b) the working volume 5, or the solar radiation can be transported from the focal plane 14 to the working volume 5 (Figures 9a, 9b, 10) via the optical transport means 8, described below. For an optimal use of this concentrator 6, the axis A must be oriented parallel to the solar rays. Therefore, this concentrator 6 is preferably dynamic, thus being able to be moved so as to assume different orientations to follow the apparent position of the Sun (Figure 3b).

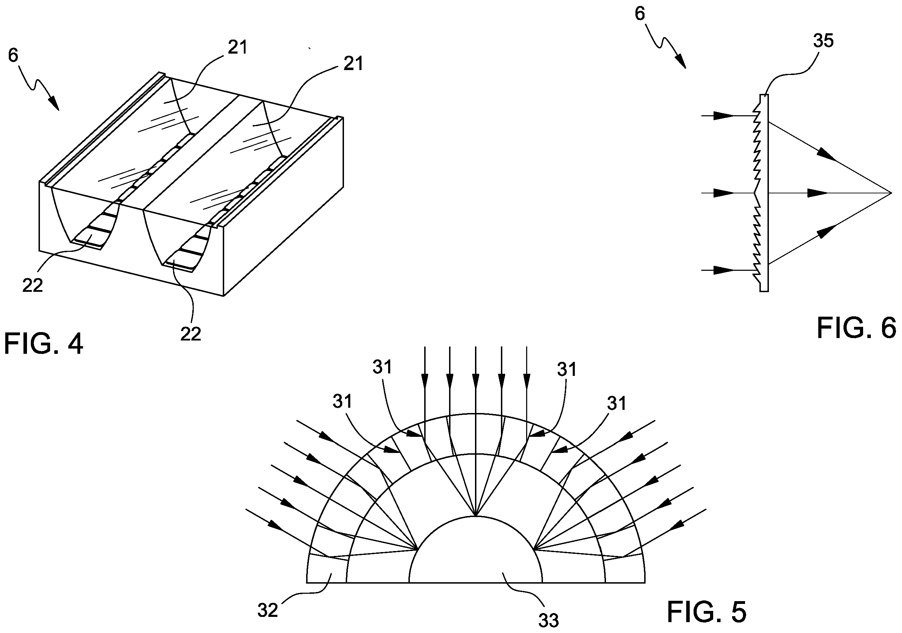

In a second embodiment (Figure 4), the concentrator 6, of parabolic type, comprises at least one mirror 21 having the shape of a parabolic cylinder, optionally truncated at a focal plane 22 thereof, which reflects and concentrates the solar rays on the focal plane 22. This concentrator 6 can have an angle of acceptance of some tens of degrees, and can thus be static.

In a third embodiment (Figure 5), the concentrator 6, of "lobster eye" type, comprises a series of flat mirrors 31, extending along radii of a spherical half-crown 32, which reflect the solar rays, irrespective of their direction, towards a semispherical detector 33. In particular, incident rays parallel to one another are reflected in a same point on the detector 33. This concentrator 6 allows to concentrate the solar rays coming from any direction of a hemisphere, and can thus be static.

According to another embodiment (Figure 6), instead of a reflection concentrator 6 as those described above, a transmission concentrator 6 may be used, for example comprising a Fresnel lens 35.

The filter 7 allows the selection of desired spectral bands and the rejection of unwanted spectral components, e.g. because they are harmful.

The choice of the desired spectral band depends on the type of application.

For example, UVC radiation, naturally present in the extra-terrestrial environment, can be used to sterilise the artificial space environment 2, as its germicidal action enables the sanitisation of air, water or surfaces.

UVB radiation, due to its greater wavelength with respect to UVC radiation, is less efficient as a germicide, thus not being used for this purpose on Earth. However, UVB radiation could be used as a germicide in the extra-terrestrial environment, since the integrated emission (power emitted per unit area) of the solar radiation in space is much higher than that on the Earth's surface, due to the filtering action of the Earth's atmosphere.

UVA radiation could be used for hydroponic cultures and/or to stimulate vitamin D production in the human body.

The visible spectrum could be used for illuminating the environment, e.g. the main environment 3.

Depending on the filter 7, it is possible to select either a narrow spectral band, e.g. comprising UVC radiation alone or UVC and UVB radiation for their germicidal action, or a broader spectral band, e.g. comprising the entire UV radiation and the visible spectrum, from which the various components can be separated later for the different applications (disinfection, hydroponics, illumination), e.g. via dichroic filters.

In general, the rejection of the harmful spectral components of solar radiation is desirable.

For example, in the UV ray band with wavelengths shorter than UVC, in particular at 185 nm, ozone, which is toxic, is formed; therefore, it is advisable to cut the spectral band lower than 220 nm.

On the other hand, considering wavelengths greater than NIR, another potentially harmful spectral band is MIR or thermal infrared, as it could cause overheating and degradation of materials or even fires, when the irradiation per unit area exceeds certain values.

In the following, the filter 7 coupled to the concentrator 6 described above in the first embodiment is described.

The filter 7 allows a transmission and/or reflection filtering.

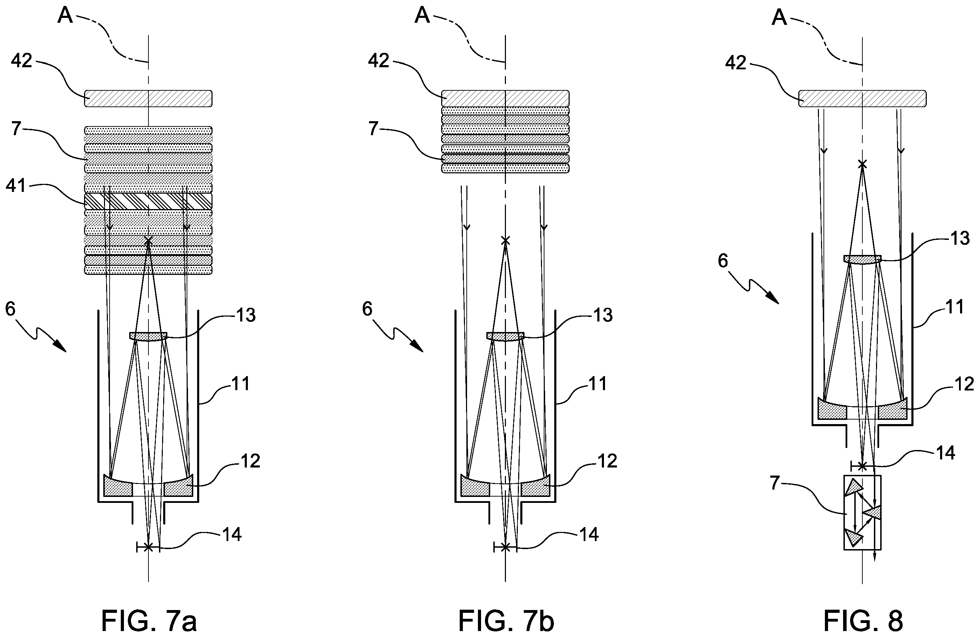

In transmission filtering (Figures 7a, 7b), the filter 7 is a bandpass interference filter arranged coaxially to the concentrator 6 and upstream of it, i.e. on the side opposite to the focal plane 14. Depending on the filter 7, it is possible to select the desired band, e.g. UVC, and eliminate the other bands. In order to increase rejection in the NIR band, a substrate 41, preferably made of glass with UG equal to 5, can be added to the filter 7. In order to abate the MIR band, it is possible to use a quartz window 42, parallel to the filter 7 which is interposed between it and the concentrator 6. In particular, the filter 7 can be spaced from the window 42 (Figure 7a), or deposited directly on it (Figure 7b).

In reflection filtering (Figure 8), the filter 7 is a multiple reflection filter system, extending substantially along the axis A, downstream of the concentrator 6. Similarly to the transmission filtering, a quartz window 42, extending orthogonally to the axis A, can be used to abate the MIR band.

In a variant (not shown), reflection filtering can be realised by depositing respective reflection filters on the primary mirror 12 and on the secondary mirror 13 of the concentrator 6.

In transmission and reflection filtering, the filter 7 comprises a substrate of high-pass material in transmission, arranged upstream of the concentrator 6, and a low-pass interference filter in reflection, arranged downstream of the concentrator 6. Therefore, a bandpass filter 7 is realised through a mixed system. Similarly to the previous cases, a quartz window 42, parallel to the transmission filter that is interposed between it and the concentrator 6, can be used to abate the MIR band.

The following are the results of simulations performed with reference to a Ritchey-Chrétien type mirror system as shown in Figures 3a, 3b, wherein the primary mirror has a diameter of 0.50 m with a central obstruction equal to one third of the diameter, the air inlet conduit has a diameter of 0.14 m, the air inlet speed is equal to 6.5 m/s and the working volume consists of a conduit with a diameter of 0.266 m and a length equal to 1 m. An integrated UVC radiation flux in the 220-280 nm band equal to 0.6 mW/cm² and an overall efficiency of the mirror and filter system equal to 0.6 is assumed, resulting in a UVC flux equal to about 0.6 W.

Assuming that the walls of the working volume have a reflectance equal to 0.95 and Lambertian scattering, a UVC radiation dose on each air element crossing the working volume equal to 15 mJ/cm² is obtained.

Using a mirror system with a double-diameter primary mirror would result in a fourfold flux and dose (equal to about 2.5 W and 60 mJ/cm², respectively).

For comparison, the average 90% inactivation of viruses and bacteria occurs with 0.6 mJ/cm², of bacterial spores with 9 mJ/cm², of fungi with 2.3 mJ/cm², of fungal spores with 31 mJ/cm² (source: Ultraviolet Germicidal Irradiation Handbook, W. Kowalsky, Springer 2009, Table 4.1).

UV radiation with a wavelength greater than 280 nm is not considered here, which however contributes, albeit with a minor effect, to the germicidal action.

If present, the optical transport means 8 (Figures 9a, 9b, 10) of the radiation allows to connect the concentrator 6 to the at least one working volume 5, in case they are distant from each other.

In Figure 9a the concentrator 6 is of the reflection type, in particular a telescope, in Figures 9b and 10 it is of the transmission type, in particular a Fresnel lens 35.

The optical transport means 8 may comprise optical fibres (Figures 9a, 9b), preferably with low absorption in the spectral bands of interest, or reflective conduits 51 (Figure 10). In particular, the reflective conduits 51 (Figure 11), preferably cylindrical, comprise internal walls 52 coated with reflective material that allow radiation to propagate by subsequent reflections. Since the characteristics of the reflective material of the internal walls 52 affect the reflection efficiency of the reflective conduits 51, it is possible to choose a coating optimised for reflecting radiation in the spectral bands of interest, so as to transport radiation over relatively long distances with low losses.

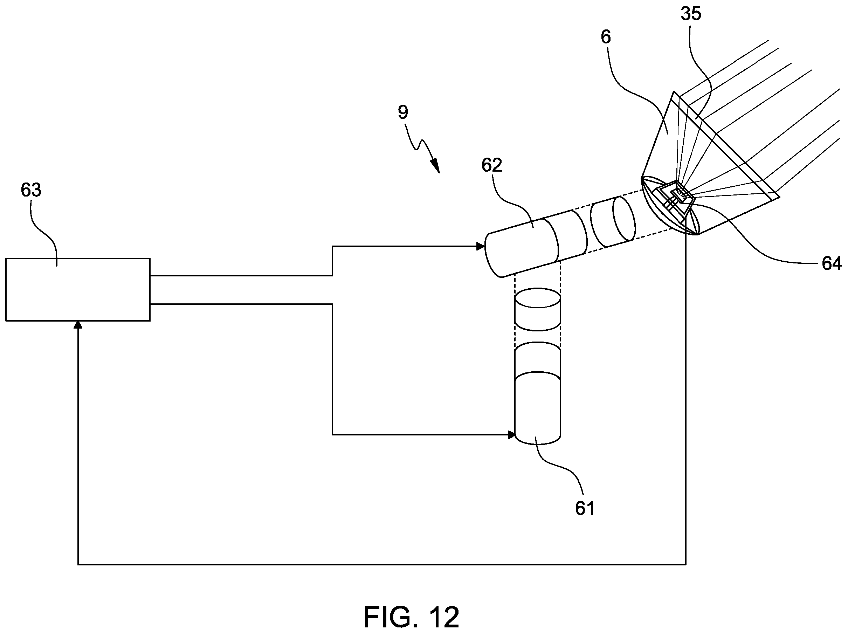

If present, the movement system 9 (Figure 12) allows to obtain a dynamic concentrator 6, which can assume a plurality of different configurations to collect solar radiation effectively.

In the following, the system 9 for moving the concentrator 6 with Fresnel lens 35 is described.

The movement system 9 allows the Sun to be followed so as to have the solar radiation always centred on the focal plane 14 of the concentrator 6.

The movement system 9 can be realised in different ways.

For example, the movement system 9 can allow an azimuth movement and a height movement, controlled by respective motors 61, 62 via a closed-loop control realised by a microprocessor 63, by a four-quadrant sensor 64 with centre on the axis A and by an electronics that measures, instant by instant, the signal difference existing between the quadrants of the sensor 64. Therefore, the control tends to align the axis A with the direction of maximum radiation.

Upon examination of the characteristics of the device 1, the advantages of the present invention are clear.

In particular, it is possible to exploit spectral bands useful for sterilisation, in particular the UVC band that is not available on Earth.

Optionally, different spectral bands may be exploited for different purposes.

If present, the optical transport means 8 of the radiation allows greater flexibility in placing the concentrator 6 and/or the at least one working volume 5.

Finally, it is clear that modifications and variations can be made to the device 1 without going beyond the scope of protection defined by the claims.

## Claims

1. Combination of artificial space environment and device, wherein the artificial space environment (2) comprises at least one working volume (5) and the device (1) is for disinfecting the at least one working volume (5), wherein the artificial space environment (2) is one of a space station, a spacecraft, a lunar base, a planetary base; wherein the device (1) is arranged externally to the artificial space environment (2) and comprises a concentrator (6) of solar radiation associated with the at least one working volume (5) for concentrating solar radiation towards the at least one working volume (5) and filtering means (7) for selecting at least one spectral band of the solar radiation comprising UVC radiation coupled to the concentrator (6).

2. Combination of artificial space environment and device as claimed in claim 1, comprising optical transport means (8) of the filtered solar radiation to connect the concentrator (6) to the at least one working volume (5).

3. Combination of artificial space environment and device as claimed in claim 2, wherein the optical transport means (8) comprise optical fibers.

4. Combination of artificial space environment and device as claimed in claim 2, wherein the optical transport means (8) comprise reflective conduits (51).

5. Combination of artificial space environment and device as claimed in any of the preceding claims, wherein the device (1) comprises means for moving (9) the concentrator (6) to maintain a direction of incidence of the solar radiation within an angle of acceptance of the concentrator (6).

6. Combination of artificial space environment and device as claimed in any of the preceding claims, wherein the concentrator (6) is a mirror system.

7. Combination of artificial space environment and device as claimed in claim 6, wherein the mirror system (6) is in a Ritchey-Chrétien optical configuration.

8. Combination of artificial space environment and device as claimed in any of claims from 1 to 5, wherein the concentrator (6) is selected from the group comprising a parabolic type concentrator, a "lobster eye" type concentrator, and a concentrator comprising a Fresnel lens (35) .

9. Combination of artificial space environment and device as claimed in any of the preceding claims, wherein the spectral band of the solar radiation comprises UVB radiation.

10. Combination of artificial space environment and device as claimed in any of the preceding claims, wherein the filtering means (7) comprise a transmission filter.

11. Combination of artificial space environment and device as claimed in any of the preceding claims, wherein the filtering means (7) comprise a reflection filter.

12. Combination of artificial space environment and device as claimed in any of the preceding claims, wherein the filtering means (7) comprise a filter (42) for rejection of an infrared band.

13. Combination of artificial space environment and device as claimed in claim 12, wherein the filter (42) comprises a quartz window.

14. Method for disinfecting at least one working volume (5) in an artificial space environment (2) using the combination as claimed in any of the preceding claims, comprising the steps of concentrating solar radiation towards the at least one working volume (5) and filtering the solar radiation to select at least one spectral band of the solar radiation comprising UVC radiation.

15. Method as claimed in claim 14, comprising the step of transporting the filtered solar radiation towards the at least one working volume (5).

## Patentansprüche

1. Kombination aus künstlicher Weltraumumgebung und Vorrichtung, wobei die künstliche Weltraumumgebung (2) mindestens ein Arbeitsvolumen (5) umfasst und die Vorrichtung (1) zum Desinfizieren des mindestens einen Arbeitsvolumens (5) dient, wobei die künstliche Weltraumumgebung (2) eine Weltraumstation, ein Raumfahrzeug, eine Mondbasis und/oder eine Planetenbasis ist; wobei die Vorrichtung (1) außerhalb der künstlichen Weltraumumgebung (2) angeordnet ist und einen dem mindestens einen Arbeitsvolumen (5) zugeordneten Sonnenstrahlung-Konzentrator (6) zum Konzentrieren der Sonnenstrahlung in Richtung des mindestens einen Arbeitsvolumens (5) und Filtermittel (7) zum Auswählen mindestens eines Spektralbandes der an den Konzentrator (6) gekoppelten UVC-Strahlung umfassenden Sonnenstrahlung umfasst.

2. Kombination aus künstlicher Weltraumumgebung und Vorrichtung nach Anspruch 1, umfassend optische Transportmittel (8) für die gefilterte Sonnenstrahlung, um den Konzentrator (6) mit dem zumindest einen Arbeitsvolumen (5) zu verbinden.

3. Kombination aus künstlicher Weltraumumgebung und Vorrichtung nach Anspruch 2, wobei die optischen Transportmittel (8) optische Fasern umfassen.

4. Kombination aus künstlicher Weltraumumgebung und Vorrichtung nach Anspruch 2, wobei die optischen Transportmittel (8) reflektierende Kanäle (51) umfassen.

5. Kombination aus künstlicher Weltraumumgebung und Vorrichtung nach einem der vorhergehenden Ansprüche, wobei die Vorrichtung (1) Mittel zum Bewegen (9) des Konzentrators (6) umfasst, um eine Einfallsrichtung der Sonnenstrahlung innerhalb eines Akzeptanzwinkels des Konzentrators (6) aufrechtzuerhalten.

6. Kombination aus künstlicher Weltraumumgebung und Vorrichtung nach einem der vorhergehenden Ansprüche, wobei der Konzentrator (6) ein Spiegelsystem ist.

7. Kombination aus künstlicher Weltraumumgebung und Vorrichtung nach Anspruch 6, wobei das Spiegelsystem (6) in einer optischen Ritchey-Chrétien-Konfiguration vorliegt.

8. Kombination aus künstlicher Weltraumumgebung und Vorrichtung nach einem der Ansprüche 1 bis 5, wobei der Konzentrator (6) aus der Gruppe ausgewählt ist, die einen Konzentrator vom Paraboltyp, einen Konzentrator vom "Hummerauge" -Typ und einen Konzentrator umfasst, der eine Fresnel-Linse (35) umfasst.

9. Kombination aus künstlicher Weltraumumgebung und Vorrichtung nach einem der vorhergehenden Ansprüche, wobei das Spektralband der Sonnenstrahlung UVB-Strahlung umfasst.

10. Kombination aus künstlicher Weltraumumgebung und Vorrichtung nach einem der vorhergehenden Ansprüche, wobei die Filtermittel (7) einen Transmissionsfilter umfassen.

11. Kombination aus künstlicher Weltraumumgebung und Vorrichtung nach einem der vorhergehenden Ansprüche, wobei die Filtermittel (7) einen Reflexionsfilter umfassen.

12. Kombination aus künstlicher Weltraumumgebung und Vorrichtung nach einem der vorhergehenden Ansprüche, wobei die Filtermittel (7) ein Filter (42) zur Unterdrückung eines Infrarotbandes umfassen.

13. Kombination aus künstlicher Weltraumumgebung und Vorrichtung nach Anspruch 12, wobei der Filter (42) ein Quarzfenster umfasst.

14. Verfahren zum Desinfizieren mindestens eines Arbeitsvolumens (5) in einer künstlichen Weltraumumgebung (2) unter Verwendung der Kombination nach einem der vorhergehenden Ansprüche, umfassend die Schritte des Konzentrierens der Sonnenstrahlung in Richtung des mindestens einen Arbeitsvolumens (5) und des Filterns der Sonnenstrahlung, um mindestens ein Spektralband der Sonnenstrahlung auszuwählen, das UVC-Strahlung umfasst.

15. Verfahren nach Anspruch 14, umfassend den Schritt des Transportierens der gefilterten Sonnenstrahlung in Richtung des mindestens einen Arbeitsvolumens (5).

## Revendications

1. Combinaison d'un environnement spatial artificiel et d'un dispositif, l'environnement spatial artificiel (2) comprenant au moins un volume de travail (5) et le dispositif (1) servant à désinfecter l'au moins un volume de travail (5), l'environnement spatial artificiel (2) étant l'un parmi une station spatiale, un vaisseau spatial, une base lunaire, une base planétaire ; le dispositif (1) étant disposé à l'extérieur de l'environnement spatial artificiel (2) et comprenant un concentrateur (6) de rayonnement solaire associé à l'au moins un volume de travail (5) pour concentrer le rayonnement solaire vers l'au moins un volume de travail (5) et des moyens de filtrage (7) pour sélectionner au moins une bande spectrale du rayonnement solaire comprenant le rayonnement UVC couplé au concentrateur (6).

2. Combinaison d'un environnement spatial artificiel et d'un dispositif selon la revendication 1, comprenant des moyens de transport optique (8) du rayonnement solaire filtré pour relier le concentrateur (6) à l'au moins un volume de travail (5).

3. Combinaison d'un environnement spatial artificiel et d'un dispositif selon la revendication 2, les moyens de transport optique (8) comprenant des fibres optiques.

4. Combinaison d'un environnement spatial artificiel et d'un dispositif selon la revendication 2, les moyens de transport optique (8) comprenant des conduits réfléchissants (51).

5. Combinaison d'un environnement spatial artificiel et d'un dispositif selon l'une quelconque des revendications précédentes, le dispositif (1) comprenant des moyens pour déplacer (9) le concentrateur (6) afin de maintenir une direction d'incidence du rayonnement solaire à l'intérieur d'un angle d'acceptation du concentrateur (6).

6. Combinaison d'un environnement spatial artificiel et d'un dispositif selon l'une quelconque des revendications précédentes, le concentrateur (6) étant un système de miroir.

7. Combinaison d'un environnement spatial artificiel et d'un dispositif selon la revendication 6, le système de miroir (6) étant dans une configuration optique Ritchey-Chrétien.

8. Combinaison d'un environnement spatial artificiel et d'un dispositif selon l'une quelconque des revendications 1 à 5, le concentrateur (6) étant choisi dans le groupe comprenant un concentrateur de type parabolique, un concentrateur de type en « œil de homard » et un concentrateur comprenant une lentille de Fresnel (35).

9. Combinaison d'un environnement spatial artificiel et d'un dispositif selon l'une quelconque des revendications précédentes, la bande spectrale du rayonnement solaire comprenant le rayonnement UVB.

10. Combinaison d'un environnement spatial artificiel et d'un dispositif selon l'une quelconque des revendications précédentes, les moyens de filtrage (7) comprenant un filtre de transmission.

11. Combinaison d'un environnement spatial artificiel et d'un dispositif selon l'une quelconque des revendications précédentes, les moyens de filtrage (7) comprenant un filtre de réflexion.

12. Combinaison d'un environnement spatial artificiel et d'un dispositif selon l'une quelconque des revendications précédentes, les moyens de filtrage (7) comprenant un filtre (42) pour le rejet d'une bande infrarouge.

13. Combinaison d'un environnement spatial artificiel et d'un dispositif selon la revendication 12, le filtre (42) comprenant une fenêtre en quartz.

14. Procédé de désinfection d'au moins un volume de travail (5) dans un environnement spatial artificiel (2) utilisant la combinaison selon l'une quelconque des revendications précédentes, comprenant les étapes de concentration du rayonnement solaire vers l'au moins un volume de travail (5) et de filtrage du rayonnement solaire pour sélectionner au moins une bande spectrale du rayonnement solaire comprenant le rayonnement UVC.

15. Procédé selon la revendication 14, comprenant l'étape de transport du rayonnement solaire filtré vers l'au moins un volume de travail (5) .
